# EUROPEAN PATENT APPLICATION

(11) **EP 3 006 005 A1**
(43) Date of publication of application: **13.04.2016**
(21) Application number: 14800290.0
(22) Date of filing: 20.05.2014
(51) Int. Cl.: A61H 1/02

(54) **TRAINING DEVICE**

(30) Priority: 24.05.2013 JP 2013110271
(71) Applicant: Kabushiki Kaisha Yaskawa Denki, Kitakyushu-shi, Fukuoka 806-0004 (JP); Kagoshima University, Kagoshima-shi, Kagoshima 890-8580 (JP)
(72) Inventor: KAWABE, Mitsunori, Kitakyushu-shi Fukuoka 806-0004 (JP); KITANO, Toshiyuki, Kitakyushu-shi Fukuoka 806-0004 (JP); MISU, Keijiro, Kitakyushu-shi Fukuoka 806-0004 (JP); SADAKANE, Ken-ichi, Kitakyushu-shi Fukuoka 806-0004 (JP); TOMISAKI, Hidenori, Kitakyushu-shi Fukuoka 806-0004 (JP); KAWAHIRA, Kazumi, Kagoshima-shi Kagoshima 890-8580 (JP); SHIMODOZONO, Megumi, Kagoshima-shi Kagoshima 890-8580 (JP); HAYASHI, Ryota, Kagoshima-shi Kagoshima 890-8580 (JP)
(74) Representative: Grünecker Patent- und Rechtsanwälte PartG mbB
(86) International application number: PCT/JP2014/063340
(87) International publication number: WO 2014/189046

(57) **Abstract**

A training device (1) is for training a limb. The training device (1) includes switches (25, 26) that are manipulated with the limb and a load reliever (3) that generates a counter force against the weight of the limb in a manner allowing the limb to move upward and downward.

## Description

### Technical Field

The disclosure relates to a device for training a limb of a patient who needs to recover a motor function.

### Background Art

A cerebral vascular disease, such as cerebral apoplexy, may result in a paralysis in a patient. To recover the motor function of a body having a partial paralysis, various devices are proposed. For example, Patent Literature 1 discloses a training device including two switches to be manipulated by an upper limb of a patient. With the training device, a patient repetitively manipulates two switches with an upper limb so that the upper limb repetitively extends and flexes. Patent Literature 2 discloses a training device including an attachment attached to an upper limb of a patient and four wires for suspending the attachment. The training device helps the motion of an upper limb by winding up or feeding out the four wires corresponding to the movement of the upper limb.

### Citation List

### Patent Literature

Patent Literature 1: JP 2006-346108 A
Patent Literature 2: JP 2012-61101 A

### Summary of Invention

### Technical Problem

However, in a training using the training device disclosed in Patent Literature 1, a patient may not have enough strength to move an upper limb to reach the switch. In such a case, an expected result may not be obtained. Meanwhile, the training device disclosed in Patent Literature 2 is configured to simultaneously wind up or feed out four wires corresponding to the motion of the upper limb, which requires many force sensors and motors. Furthermore, cooperation of the force sensors and the motors requires complicated control. Therefore, the apparatus might become complex and expensive.

An object of the disclosure is to provide an apparatus that has simple configuration and can provide effective training.

### Solution to Problem

A training device according to the disclosure is for training a limb and includes a switch that is manipulated with the limb and a load reliever that generates a counter force against the weight of the limb in a manner allowing the limb to move upward and downward.

### Advantageous Effects of Invention

According to the disclosure, an effective training can be conducted with a simple configuration.

### Brief Description of Drawings

FIG 1 is a perspective view of a training device.
FIG 2 is a side view illustrating an adjustor for adjusting a position of a switch.
FIG 3 is a perspective view illustrating an exemplary modification of a chest stopper.
FIG. 4 is a schematic view illustrating a training.

### Description of Embodiments

An embodiment will be described in detail referring to the attached drawings. In the description, the same component or the component having the same function is denoted with the same reference sign and repeated description thereof will be omitted.

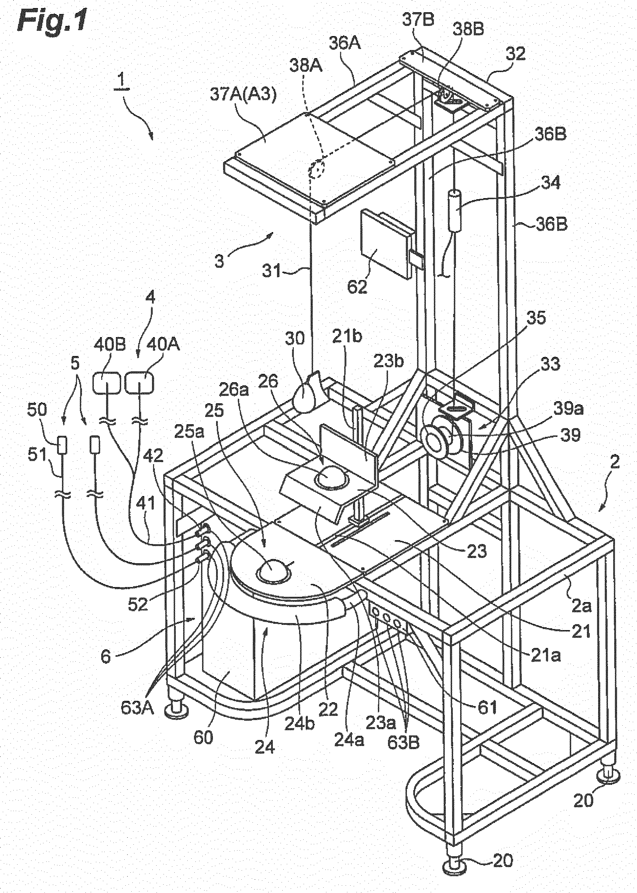

As illustrated in FIG 1, a training device 1 according to an embodiment is for training an upper limb of a patient who needs to recover a motor function. A patient who needs to recover a motor function may be a patient who has a partial paralysis in the body resulting from a cerebral vascular disease, for example, cerebral apoplexy. The training device 1 includes a work platform 2, a load reliever 3, an electrical stimulator 4, a vibratory stimulator 5, and a controller 6.

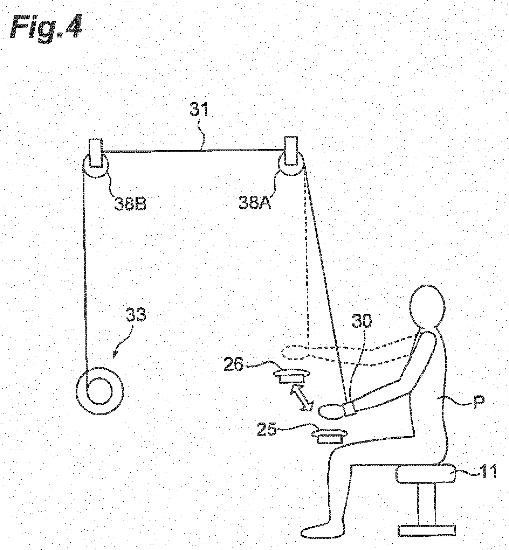

The work platform 2 is placed on the floor. A chair 11 (see FIG. 4) for a patient is placed near the work platform 2. Hereinafter in the description, terms "forward", "rearward", "left", and "right" indicates directions, where the direction toward a patient is the rear direction and the direction remote from a patient is the forward direction. The work platform 2 is configured with, for example, an aluminum frame, and has an approximately cuboid external profile. The long sides of the work platform 2 extend along the right and left direction. A leg 20 is provided on each of four corners on the bottom of the work platform 2.

The upper portion 2a of the work platform 2 is at a height where the chest of a patient sitting on the chair 11 comes. The upper portion 2a is provided with a top plate 21, a first support plate 22, a second support plate 23, and a chest stopper 24. The top plate 21 is horizontally positioned in the middle in the right and left direction of the work platform 2 and close to the rear edge of the work platform 2.

The first support plate 22 is positioned adjacent the rear side of the top plate 21 to protrude from the rear edge of the work platform 2 in a tongue-shape. A first switch 25 to be manipulated by an upper limb of a patient is provided on the first support plate 22. That is, the training device 1 includes the first switch 25. The first switch 25 has a dome-shaped push button 25a. By pushing the push button 25a, the first switch 25 is switched on or off.

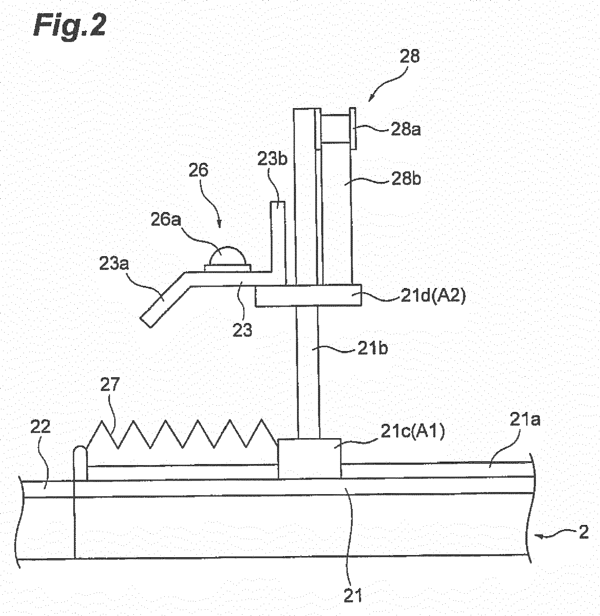

As illustrated in FIGS. 1 and 2, the top plate 21 is provided with a guide 21a extending along the forward and rearward direction and a forward/rearward slider 21c mounted on the guide 21a. The position of the forward/rearward slider 21 c can be changed along the guide 21a. The forward/rearward slider 21c is provided with a vertical strut 21b and an upward/downward slider 21d mounted on the strut 21b. The position of the upward/downward slider 21d can be changed along the strut 21 b.

The second support plate 23 is attached to the upward/downward slider 21d, protrudes rearward, and faces the top plate 21. A second switch 26 to be manipulated by an upper limb of a patient is provided on the second support plate 23. That is, the training device 1 includes the second switch 26. The second switch 26 has a dome-shaped push button 26a. By pushing down the push button 26a, the second switch 26 is switched on or off. The second switch 26 is positioned in the forward side of the first switch 25 and higher than the first switch 25.

A tilt portion 23a which become lower toward the rearward side is provided on the rear portion of the second support plate 23. The tilt portion 23a allows a patient to push the push button 26a of the second switch 26 with an upper limb with little chance of interference between the upper limb and the rear portion of the second support plate 23. A vertical wall 23b is vertically provided on the forward portion of the second support plate 23. The vertical wall 23b prevents an upper limb of a patient from moving excessively forward to fall off from the second support plate 23.

Positions of the second support plate 23 and the second switch 26 can be adjusted along the forward and rearward direction by changing the position of the forward/rearward slider 21c. That is, the forward/rearward slider 21c constitutes an adjustor A1 for adjusting the position of the second switch 26 along the forward and rearward direction. Heights of the second support plate 23 and the second switch 26 can be adjusted by changing the position of the upward/downward slider 21d. That is, the upward/downward slider 21 d constitutes an adjustor A2 for adjusting the height of the second switch 26. The training device 1 includes adjustors A1 and A2 to adjust the position of the second switch 26 along the forward and rearward direction and the upward and downward direction.

A bellows cover 27 is provided over the guide 21a in a region in the rearward side of the forward/rearward slider 21c. The rear end of the cover 27 is fixed to the rear end of the guide 21 a, and the front end of the cover 27 is fixed to the forward/rearward slider 21c. The cover 27 extends and contracts along with the change in the position of the forward/rearward slider 21c. The cover 27 prevents an upper limb of a patient from touching the guide 21 a.

An anti-drop stopper 28 that generates a counter force against the descending of the second switch 26 while adjusting the height is provided on the upper end of the strut 21b. The anti-drop stopper 28 includes a winding up shaft 28a protruding forward from the strut 21b and a sheet spring 28b wound around the winding up shaft 28a. An end of the sheet spring 28b is fixed to the upward/downward slider 21d. The sheet spring 28b is fed out from the winding up shaft 28a along with the downward movement of the upward/downward slider 21 d, generating a counter force against the descending of the upward/downward slider 21 d. In this manner, the weight of the second switch 26 and its support members (the second support plate 23 and the upward/downward slider 21 d) is reduced, which makes it easy to adjust the height of the second switch 26.

The chest stopper 24 includes a chest stopping frame 24a provided along the rim of the first support plate 22 and a cushion 24b covering the chest stopping frame 24a. Both the ends of the chest stopping frame 24a are fixed to the upper portion 2a of the work platform 2. The chest stopper 24 restricts the movement of the chest of a patient toward the switches 25 and 26. When manipulating the switches 25 and 26, the movement of the chest toward the switches 25 and 26 is restricted, so that an upper limb has to be moved further. With the restriction on the movement of the chest, a larger amount of exercise is required of an upper limb.

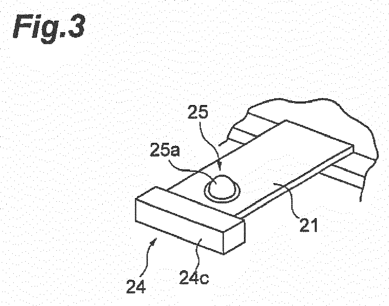

As illustrated in FIG. 3, the first support plate 22 may have a rectangular shape with the long sides along the forward and rearward direction. The cushion 24c may be provided only on the short side close to a patient of the first support plate 22 to constitute the chest stopper 24. In such a configuration, the area occupied by the first support plate 22 and the chest stopper 24 is small in size along the right and left direction, so that the motion of an upper limb of a patient who cannot lift up an elbow is not hindered.

As illustrated in FIG. 1, the load reliever 3 includes a sling 30, a wire 31, a wire guide 32, a tensioner 33, a tension sensor 34, and a tension adjustor 35. The sling 30 is an attachment to be attached to a wrist of a patient which has a form of a belt to surround a wrist. The wire 31 is connected to the sling 30 pulled upward from the sling 30.

The wire guide 32 includes a frame body 36A, two connecting frames 36B, two top plates 37A and 37B, and two pulleys 38A and 38B. The frame body 36A is configured with, for example, a rectangular aluminum frame. The frame body 36A is horizontally positioned above the work platform 2 with the long sides along the forward and rearward direction. The connecting frames 36B are, for example, vertically extending aluminum frames disposed side by side along the right and left direction. The connecting frames 36B connect the front edge of the frame body 36A and the front edge of the work platform 2.

The top plates 37A and 37B are each provided over the upper portion of the frame body 36A. The top plate 37A is positioned close to the rear edge of the frame body 36A, and the top plate 37B is positioned close to the front edge of the frame body 36A. The pulley 38A is attached to the middle of the bottom of the top plate 37A and higher than the sling 30. The position where the pulley 38A is attached to the top plate 37A can be adjusted along the forward and rearward direction. That is, the load reliever 3 includes an adjustor A3 for adjusting the position of the pulley 38A along the forward and rearward direction. The pulley 38B is attached to the middle of the bottom of the top plate 37B.

The wire 31 pulled upward from the sling 30 runs about the pulley 38A to be directed forward and runs about the pulley 38B to be directed downward. The front end of the wire 31 running about the pulley 38B to be directed downward is connected to the tensioner 33.

The tensioner 33 is fixed to the bottom ends of the connecting frames 36B and applies a tensional force to the wire 31 in a manner allowing the sling 30 to move upward and downward. Specifically, the tensioner 33 includes a constant load spring element 39 as a passive element. The constant load spring element 39 generates a counterbalancing force to apply a tensional force to the wire 31. That is, the tensioner 33 applies a tensional force to the wire 31 by the elastic energy stored in the constant load spring element 39. The passive element is an element that only functions when receiving an external force.

The constant load spring element 39 includes a reel 39a rotatable about an axis along the forward and rearward direction and embedded with a coiled sheet spring generating a force against the rotation of the reel 39a. The front portion of the wire 31 is wound around the reel 39a. The constant load spring element 39 applies an approximately constant tensional force to the wire 31 with a counterbalancing force from the sheet spring and simultaneously winds up or feeds out the wire 31 corresponding to the upward and downward movement of the sling 30.

The passive element that can be used for the tensioner 33 is not limited to the constant load spring element 39. Any passive element that can store potential energy and use the potential energy to apply a tensional force to the wire 31 can be used. For example, the passive element may be a linear spring element that extends and contracts corresponding to the upward and downward movement of the sling 30. In this case, a tensional force can be applied to the wire 31 by the elastic energy stored in the linear spring element. Alternatively, the passive element may be a weight. In this case, a tensional force can be applied to the wire 31 by the potential energy stored in the weight.

The passive element may not be used in the tensioner 33. For example, an electric motor for winding up the wire 31 may be used, so that a tensional force can be applied to the wire 31 by controlling the torque of the electric motor.

The tensional force applied to the wire 31 by the tensioner 33 serves as a counter force against the weight of an upper limb of a patient to which the sling 30 is attached. That is, the load reliever 3 generates a counter force against the weight of an upper limb of a patient in a manner allowing the upper limb to move upward and downward.

The tension sensor 34 is embedded with, for example, a load cell to detect a tensional force applied to the wire 31. The tension sensor 34 is provided on the wire 31 at a location between the pulley 38B and the constant load spring element 39.

The tension adjustor 35 adjusts the tensional force applied to the wire 31 by changing the location where the sheet spring is fixed in the constant load spring element 39. The tension adjustor 35 is adjacent the rear side of the constant load spring element 39 and faces the patient. Thus the tensional force can easily be adjusted from the patient side.

The electrical stimulator 4 includes a pair of flexible sheet electrodes 40A and 40B and a power feeding cable 41 connected to both the electrodes 40A and 40B. The electrodes 40A and 40B are stuck on portions of an upper limb of a patient where the motion during the training is related to. The electrical stimulator 4 is supplied with power via the power feeding cable 41 and generates a current across the electrodes 40A and 40B to give an electrical stimulus to a muscle of a patient. A connector 42 is provided on the end opposite the electrodes 40A and 40B of the power feeding cable 41.

The vibratory stimulator 5 includes, for example, a vibrating body 50 embedded with a vibration motor and a power feeding cable 51 connected to the vibrating body 50. By using an adhesive tape or the like, the vibrating body 50 is stuck on a portion of an upper limb of a patient where the motion during the training is related to. The vibratory stimulator 5 is supplied with power via the power feeding cable 51 and gives a vibratory stimulus from the vibrating body 50 to an upper limb of a patient. A connector 52 is provided on the end opposite the vibrating body 50 of the power feeding cable 51.

The number of the electrical stimulator 4 and the number of the vibratory stimulator 5 are not limited. Each number may be one or more. FIG 1 illustrates a case where one electrical stimulator 4 and two vibratory stimulators 5 are provided.

The controller 6 includes a main body 60, a terminal 61, and a monitor 62 and controls the electrical stimulator 4 and the vibratory stimulator 5. The main body 60 is embedded with a controlling computer and disposed in the left portion of the work platform 2. A plurality of connectors 63A connected to the controlling computer is provided on the upper portion of the rear face (the face close to a patient) of the main body 60. A connector 42 of the electrical stimulator 4 or a connector 52 of the vibratory stimulator 5 is connected to the connector 63A. In this manner, the electrical stimulator 4 and the vibratory stimulator 5 are connected to the controlling computer in the main body 60. The switches 25 and 26, the tension sensor 34, and the monitor 62 are also connected to the controlling computer in the main body 60 via cables (not shown).

The terminal 61 is a connector unit including a plurality of connectors 63B. The connector 63B is same as the connector 63A. The connector 63B is provided on the rear face (the face close to a patient) of the terminal 61. The terminal 61 is fixed to the right portion of the work platform. That is, when viewed from a patient, the switches 25 and 26 are provided between the main body 60 and the terminal 61.

The connectors 63B are connected to the controlling computer in the main body 60 via cables (not shown) and arrayed in parallel to the connector 63A of the main body 60 with regard to the controlling computer. Thus, in a similar manner as the connection to the connector 63A, the electrical stimulator 4 and the vibratory stimulator 5 can be connected to the controlling computer by connecting the connector 42 and the connector 52 to the connector 63B. In this manner, the electrical stimulator 4 and the vibratory stimulator 5 can selectively be connected to either right or left side to the switches 25 and 26 according to whether training is performed for the right upper limb or the left upper limb.

The monitor 62 is, for example, a liquid crystal display fixed to a connecting frame 36B in a manner facing a patient. The monitor 62 may be a touch panel that can be used as an input device to the controlling computer.

The controller 6 supplies power via the power feeding cables 41 and 51 to drive the electrical stimulator 4 and the vibratory stimulator 5. The power supplied to the electrical stimulator 4 can previously be set using an input device, such as a key board (not shown). The timing of supplying power to the vibratory stimulator 5 can also be set using the input device, such as a key board (not shown).

As an example of a setting of the timing of driving the vibratory stimulator 5, the vibratory stimulator 5 may be driven in response to the on and off of the switches 25 and 26. In such a setting, the vibratory stimulator 5 gives a vibratory stimulus to an upper limb in response to the on and off of the switches 25 and 26. For example, the timing may be set such that the driving starts when one of the switches 25 and 26 is pushed and the driving stops when the other one of the switches 25 and 26 is pushed. The setting of the timing may be such that a plurality of vibratory stimulators 5 can be driven at different timings. The setting of the timing may be such that the vibratory stimulator 5 can continuously be driven during the training or the vibratory stimulator 5 cannot be driven throughout the training.

The setting of the timing of driving the electrical stimulator 4 as well as the setting of the timing of driving the vibratory stimulator 5 may be allowed. As an example of a setting of the timing of driving the electrical stimulator 4, the electrical stimulator 4 may be driven in response to the on and off of the switches 25 and 26. In such a setting, the electrical stimulator 54 gives an electrical stimulus to an upper limb in response to the on and off of the switches 25 and 26. For example, the timing may be set such that the driving starts when one of the switches 25 and 26 is pushed and the driving stops when the other one of the switches 25 and 26 is pushed. The timing may be set such that the electrical stimulator 4 can continuously be driven during the training or the electrical stimulator 4 cannot be driven throughout the training.

The controller 6 presents various kinds of information related to the training on the monitor 62. The information to be presented includes, for example, the numbers of on and off of the switches 25 and 26, a time interval between on and off of the switches 25 and 26, and a tensional force detected by the tension sensor 34.

The procedure of training using the training device 1 will now be described. A patient P first sits on the chair 11 in the rearward side of the work platform 2 as illustrated in FIG 4. The sling 30 is attached to a wrist of the patient P. The electrodes 40A and 40B of the electrical stimulator 4 and the vibrating body 50 of the vibratory stimulator 5 are attached to portions of an upper limb of the patient P where the motion is related to.

The counter force generated by the load reliever 3 is adjusted with the tension adjustor 35 according to the weight of the upper limb of the patient P. The counter force is set within the weight of the upper limb of the patient P such that the patient P can support the upper limb by his or her muscle power. When adjusting the counter force, the tension value detected by the tension sensor 34 is presented on the monitor 62 to be checked. By checking the tension value, the weight of the upper limb of the patient P can accurately be checked to set an appropriate condition according to the weight of the upper limb.

The current value to be supplied from the controller 6 to the electrical stimulator 4 is set. The current value is set such that the joint of the upper limb does not move by an electrical stimulus. Then, the timing to supply power from the controller 6 to the vibratory stimulator 5 is set. The preparation for training is now complete. The sequential order of attaching the sling 30, sticking the electrodes 40A and 40B, sticking the vibrating body 50, and conducting various settings is not limited to the order described above.

Now, the patient P performs a repetitive motion, namely, alternately pushing the switches 25 and 26, to train the upper limb of the patient P. A set of the training finishes when the number of the repetitive motions reaches a target number. A set of the training may be finished when a previously determined time has elapsed.

During the repetitive motion, the load reliever 3 generates a counter force against the weight of the upper limb in a manner allowing the upper limb to move upward and downward. Thus the weight of the upper limb is continuously reduced during the repetitive motion. The load reliever 3 is required to generate only a counter force against the weight, so that the configuration of the load reliever 3 can be simplified. For example, a group of the sling 30, the wire 31, and the tensioner 33 constitutes the load reliever 3 as described above. Since the motion required of the upper limb is only a simple motion of pushing the switches 25 and 26, the easiness of training can drastically be improved by continuously reducing the weight with the load reliever 3. Since the load reliever 3 does not force the upper limb to move, the patient P extends and flexes the upper limb by his or her own strength. So that the training is highly effective for recovering the motor function of the upper limb. Thus, an effective training can be performed with a simple configuration.

The load reliever 3 generates a counter force against the weight of the upper limb only by the tensional force applied to the single wire 31 in a manner allowing the upper limb to move upward and downward. Since this configuration hardly restricts the position of the upper limb, the motion is performed further by the strength of the patient P.

The tensioner 33 of the load reliever 3 applies a tensional force to the wire 31 by the elastic energy stored in the constant load spring element 39 used as a passive element. When a weight is used as the passive element of the tensioner 33 to apply a tensional force to the wire by the gravitational potential energy of the weight, the inertial force of the weight generated by acceleration and deceleration of the upper limb is likely to cause rapid fluctuation of the tensional force. In contrast, the use of the elastic energy of the spring element reduces the effect of the inertial force and thus suppresses the rapid fluctuation of tensional force. In particular, by using the constant load spring element 39 as the spring element, the fluctuation of tensional force corresponding to the position of the upper limb can also be suppressed. As a result, the weight of the upper limb can be reduced by a stable counter force.

During the repetitive motion, the electrical stimulator 4 gives an electrical stimulus to the upper limb of the patient P. Stimulating the muscle of the patient P in this manner can further improve the easiness of training. Since a current value given to the electrical stimulator 4 is set so as not to generate a motion of a joint, the upper limb is not forced to move. Thus the effect of facilitating the motion by the strength of the patient P is not deteriorated.

During the repetitive motion, the vibratory stimulator 5 gives a vibratory stimulus to the upper limb of the patient P. A vibratory stimulus effectively gives effect on deep sensitivity of a muscle of the patient P and stimulates a nerve pathway from the cerebrum to the muscle. Thus the motor function can be recovered effectively. In particular, when the vibratory stimulator 5 is driven in response to the manipulation of the switches 25 and 26, the stimulation to a nerve pathway is repeated corresponding to the repetitive motion of the upper limb. This further effectively facilitates the recovery of the motor function.

As described above, the training device 1 includes the adjustors A1 and A2 for adjusting the position of the second switch 26. With the adjustor A2, the relative position along the upward and downward direction of the second switch 26 to the first switch 25 can be adjusted. So that the height difference between the first switch 25 and the second switch 26 can be adjusted considering the degree of paralysis or the degree of recovery of motor function of the patient P. For example, if the degree of paralysis is low, the height difference between the first switch 25 and the second switch 26 may be increased to raise the load of the training. As the motor function recovers by repeating the training, the height difference between the first switch 25 and the second switch 26 may be increased to raise the load of the training.

Furthermore, with the adjustor A1, the relative position along the forward and rearward direction of the second switch 26 to the first switch 25 can be adjusted. Considering the degree of paralysis or the degree of recovery of motor function of the patient P, the distance along the forward and rearward direction between the first switch 25 and the second switch 26 can be adjusted. For example, if the degree of paralysis is low, the distance along the forward and rearward direction between the first switch 25 and the second switch 26 may be lengthened to increase the moving distance of the upper limb. As the motor function recovers by repeating the training, the distance along the forward and rearward direction between the first switch 25 and the second switch 26 may be lengthened to increase the moving distance of the upper limb.

The easiness of training can be controlled by adjusting the position of the second switch 26 using the adjustors A1 and A2, so that a further effective training can be performed. The adjustors A1 and A2 may be configured to adjust the position of the first switch 25 instead of the second switch 26 or configured to adjust both the positions of the first switch 25 and the second switch 26.

The training device 1 includes an adjustor A3 for adjusting the position of the pulley 38A along the forward and rearward direction. With this mechanism, the position of the sling 30 suspended from the pulley 38A can be adjusted considering the positions of the first switch 25 and the second switch 26 so that the repetitive motion can be performed more smoothly.

A tensional force applied to the wire 31, that is, a counter force generated by the load reliever 3 may be adjusted according to the degree of paralysis or the degree of recovery of motor function of the patient P. For example, if the degree of paralysis is low, the counter force may be reduced to raise the load of the training. As the recovery of motor function progresses, the counter force may be reduced to raise the load of training.

The controller 6 may store training data. The training data includes a cycle period of the repetitive motion and a tensional force applied to the wire 31. Such training data may be analyzed afterward to check the degree of recovery of motor function. For example, the progress of recovery of motor function can be checked from the decrease in a cycle period of the repetitive motion.

The scope of the present invention is not particularly limited to the embodiment described above. Various modifications can be made without departing from the scope and spirit of the invention. For example, the electrical stimulator 4 and the vibratory stimulator 5 are not necessarily included in the training device. The number of switches to be manipulated by an upper limb of a patient is not limited to two. The number of switch or switches may be one, or three or more. The present invention can also be applied to the training of a lower limb of a patient. That is, the present invention can be applied to the training of limbs including an upper limb and a lower limb.

### Industrial Applicability

The disclosure can be used for a device for training a limb of a patient who needs to recover a motor function.

### Reference Signs List

- 1: training device
- 3: load reliever
- 5: vibratory stimulator
- 25: first switch
- 26: second switch
- 30: sling (attachment)
- 31: wire
- 33: tensioner
- 34: tension sensor
- 35: tension adjustor
- 39: constant load spring element (passive element)
- A1, A2: adjustor for adjusting a position of the switch

## Claims

1. A training device for training a limb, the training device comprising:
a switch being manipulated with the limb; and
a load reliever generating a counter force against a weight of the limb in a manner allowing the limb to move upward and downward.

2. The training device according to claim 1, further comprising a vibratory stimulator giving a vibratory stimulus to the limb in response to manipulation of the switch.

3. The training device according to claim 1 or 2, further comprising an electrical stimulator that gives an electrical stimulus to the limb with a current value not generating a motion of a joint.

4. The training device according to claim 3, wherein the electrical stimulator gives the electrical stimulus to the limb in response to manipulation of the switch.

5. The training device according to any one of claims 1 to 4, wherein the load reliever includes
an attachment to be attached to the limb,
a wire pulled upward from the attachment, and
a tensioner that generates a counter force against the weight by applying a tensional force to the wire in a manner allowing the attachment to move upward and downward.

6. The training device according to claim 5, wherein the tensioner applies the tensional force by potential energy stored in a passive element.

7. The training device according to claim 6, wherein the tensioner applies the tensional force by elastic energy stored in a spring element.

8. The training device according to claim 7, wherein the tensioner applies the tensional force by elastic energy stored in a constant load spring element.

9. The training device according to any one of claims 5 to 8, wherein the load reliever includes a tension sensor for the wire, and an adjustor for the tensional force.

10. The training device according to any one of claims 1 to 9, further comprising an adjustor for adjusting a position of the switch.

11. The training device according to claim 10, further comprising an anti-drop stopper, wherein
the adjustor is an adjustor for adjusting a height of the switch, and
the anti-drop stopper generates a counter force against descending of the switch when adjusting the height.
